(19) Europäisches Patentamt / European Patent Office / Office européen des brevets

(11) **EP 4 603 182 A1**

(12) **EUROPEAN PATENT APPLICATION**
published in accordance with Art. 153(4) EPC

(43) Date of publication:
**20.08.2025   Bulletin 2025/34**

(21) Application number: **23876484.9**

(22) Date of filing: **20.09.2023**

(51) International Patent Classification (IPC):
**B01J 23/44** (2006.01)     **B01J 23/50** (2006.01)
**B01J 23/54** (2006.01)     **B01J 23/58** (2006.01)
**B01J 23/60** (2006.01)     **B01J 23/62** (2006.01)
**B01J 37/02** (2006.01)     **C07C 5/09** (2006.01)
**C07C 7/167** (2006.01)     **C07C 11/04** (2006.01)
**C07C 11/06** (2006.01)

(52) Cooperative Patent Classification (CPC):
**B01J 23/44; B01J 23/50; B01J 23/54; B01J 23/58;
B01J 23/60; B01J 23/62; B01J 27/138;
B01J 27/232; B01J 31/02; B01J 31/04;
B01J 37/02; C07C 5/05; C07C 5/09;** C07C 7/167;
C07C 11/04;                              (Cont.)

(86) International application number:
**PCT/CN2023/119966**

(87) International publication number:
**WO 2024/078279 (18.04.2024 Gazette 2024/16)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **14.10.2022   CN 202211261295**

(71) Applicants:
• **China Petroleum & Chemical Corporation
  Beijing 100728 (CN)**
• **Sinopec (Beijing) Research Institute of
  Chemical Industry Co., Ltd.
  Beijing 100013 (CN)**

(72) Inventors:
• **WEI, Guobin
  Beijing 100013 (CN)**
• **PENG, Hui
  Beijing 100013 (CN)**
• **TIE, Kai
  Beijing 100013 (CN)**
• **ZHANG, Liyan
  Beijing 100013 (CN)**
• **MU, Wei
  Beijing 100013 (CN)**
• **YANG, Chenxi
  Beijing 100013 (CN)**
• **YI, Shuisheng
  Beijing 100013 (CN)**
• **LU, Hongliang
  Beijing 100013 (CN)**

(74) Representative: **karo IP
Patentanwälte PartG mbB
Steinstraße 16-18
40212 Düsseldorf (DE)**

(54) **SUPPORTED PALLADIUM CATALYST, AND PREPARATION AND USE THEREOF**

(57)     Disclosed are a supported palladium catalyst, and preparation and use thereof, the catalyst comprising a support, and palladium and a modified component supported on the support, wherein the support is selected from refractory metal oxides, silicon oxide, activated carbon, or combinations thereof, and the modified component is selected from Bi, Sb, Pb, Sn, Zn, W, Mn, Si, Re, Group VIII elements other than palladium, alkali metal elements, alkaline earth metal elements, Group IIIA elements, Group IB elements, rare earth elements, halogen elements, or combinations thereof, wherein according to an analysis on in-situ infrared spectroscopy of pyrrole adsorption carried out at 40°C, the catalyst exhibits absorption peak(s) in the range of 3251-3410 cm$^{-1}$. The supported palladium catalyst features an overall weakly acidic surface environment with specific weakly basic sites, resulting in better catalytic performance of the catalyst.

EP 4 603 182 A1

FIG.2

(52) Cooperative Patent Classification (CPC): (Cont.)
    C07C 11/06

**Description**

## TECHNICAL FIELD

The present application relates to the field of catalyst, and in particular to a supported palladium catalyst, and preparation and use thereof.

## BACKGROUND ART

Palladium (Pd)-based catalysts have been widely used in the petrochemical industry; for example, catalytic reforming, isomerization and dehydrogenation of alkanes and aromatics, selective hydrogenation in olefin production, and the production of organic chemical raw materials such as acetaldehyde, vinyl acetate, and methyl methacrylate all rely on palladium-based catalysts. In addition, palladium serves as an excellent catalyst or one of the important components of the catalyst in various organic chemical reactions such as hydrogenation, oxidative dehydrogenation, hydrocracking, coupling, hydroesterification, C1 chemistry, and automobile exhaust purification.

Since pure noble metal catalysts suffer from drawbacks such as high cost, narrow active temperature ranges, and susceptibility to deactivation in the presence of oxygen, severely limiting their application, the development of palladium-based noble metal catalysts has primarily focused on supported catalysts. For example, CN113067000A discloses a Pd-Co nanoalloy catalyst supported on $TiO_2$ with oxygen vacancies. Palladium is dispersed onto suitable supports for use by using methods such as impregnation, metal ion vapor deposition, solvated metal atom impregnation, ion exchange and sol-gel method. For different reactions, suitable supports are used. For example, CN112517063A discloses a method for preparing a Pd-Au in elemental state supported catalyst for vinyl acetate production via the ethylene process, where the catalyst is then prepared by impregnating chloropalladic acid and chloroauric acid onto a silica support, then adding a surfactant and an aqueous solution of sodium silicate to precipitate palladium and gold compounds, subjecting to hydrogen reduction to elemental state and impregnating with potassium acetate solution, which effectively improves the activity and selectivity of the catalyst.

The surface acidity or alkalinity of the support directly affects the degree of "metal-support interaction" on the catalyst surface and the dispersion state of the active metal, which in turn determines the activity and selectivity of the catalyst. The role of acidic or basic sites often enables many reactions to proceed smoothly, such as certain hydrogenations, hydrogen transfers, aldol condensation reactions, etc. Coking is often a troublesome problem in acidic-catalyzed hydrogenation reactions, while alkaline catalysts typically avoid coking in hydrogenation reactions but are prone to deactivation at higher alkalinity. Therefore, there is an urgent need in the field to develop a supported palladium-based catalyst with good catalytic effect.

## SUMMARY OF INVENTION

The objective of the present application is to provide a supported palladium catalyst, and preparation and use thereof. The supported palladium catalyst features an overall weakly acidic surface environment with specific weakly basic sites, resulting in better catalytic performance of the catalyst.

In order to achieve the above-mentioned objective, in one aspect, the present application provides a supported palladium catalyst, comprising a support, and palladium and a modifying component supported on the support, wherein the support is selected from refractory metal oxides, silicon oxide, activated carbon, or combinations thereof, and the modifying component is selected from Bi, Sb, Pb, Sn, Zn, W, Mn, Si, Re, Group VIII elements other than palladium, alkali metal elements, alkaline earth metal elements, Group IIIA elements, Group IB elements, rare earth elements, halogen elements, or combinations thereof, wherein according to an analysis on in-situ infrared spectroscopy of pyrrole adsorption carried out at 40°C, the catalyst exhibits absorption peak(s) in the range of 3251-3410 $cm^{-1}$.

Preferably, according to the analysis on in-situ infrared spectroscopy of pyrrole adsorption carried out at 40°C, the ratio of the peak height of the absorption peak in the range of 3251-3410 $cm^{-1}$ in the in-situ infrared spectrum of the catalyst measured when purging with nitrogen is carried out for 0 minute after 10 minutes of pyrrole adsorption to the peak height of the corresponding absorption peak in the range of 3251-3410 $cm^{-1}$ in the in-situ infrared spectrum measured when purging with nitrogen is carried out for 15 minutes is more than or equal to 5: 1.

In another aspect, provided is a method for preparing the supported palladium catalyst of the present application, comprising the following steps:

  1) loading palladium and a modifying component onto a support, followed by drying and calcining to obtain an intermediate; and
  2) subjecting the intermediate to an alkalization treatment with an alkaline solution having a pH value of 7.2-10, followed by drying and optionally calcining to obtain the catalyst,

wherein the alkalization treatment is achieved by spraying the alkaline solution, the spraying time is 2-8 min, and the spraying temperature is 35-70°C.

In still another aspect, provided is use of the supported palladium catalyst of the present application in hydrogenation of unsaturated hydrocarbon, wherein the unsaturated hydrocarbon is selected from alkynes, polyunsaturated hydrocarbons, or combinations thereof.

In yet another aspect, provided is a method for hydrogenating unsaturated hydrocarbon, comprising a step of contacting an unsaturated hydrocarbon feedstock comprising alkyne and/or polyunsaturated hydrocarbon with the supported palladium catalyst of the present application in the presence of hydrogen for reaction.

The supported palladium catalyst of the present application exhibits absorption peak(s) in the range of 3251-3410 $cm^{-1}$ in the in-situ infrared spectrum of pyrrole adsorption measured at 40°C, indicating the presence of specific weakly basic sites on the surface of the catalyst. The supported palladium catalyst of the present application features an overall weakly acidic surface, with also specific weakly basic sites at the same time, so that when the catalyst is used for hydrogenation of unsaturated hydrocarbon (especially selective hydrogenation of alkyne and polyunsaturated hydrocarbon), it has better catalytic activity and selectivity than existing catalysts.

In the method for preparing the catalyst of the present application, an alkaline solution is used to alkalize the intermediate loaded with palladium and the modified component under specific conditions, providing specific weakly basic sites for the obtained catalyst. The catalyst exhibits absorption peak(s) in the range of 3251-3410 $cm^{-1}$ in the in-situ infrared spectrum of pyrrole adsorption measured at 40°C, thereby enabling it to have better catalytic activity and selectivity when used for hydrogenation of unsaturated hydrocarbon (especially selective hydrogenation of alkyne and polyunsaturated hydrocarbon). Moreover, the preparation method is simple and easy to implement, facilitating industrial production.

Other features and advantages of the present application will be described in detail in the subsequent specific implementation section.

## DESCRIPTION OF DRAWINGS

The accompanying drawings are used to provide a further understanding of the present application and constitute a part of the specification. Together with the following specific implementation, they are used to explain the present application but do not constitute a limitation to the present application. In the accompanying drawings:

FIG. 1A to 1J show the in-situ infrared spectra of pyrrole adsorption of catalysts A-J prepared in Examples and Comparative Examples; and
FIG. 2 shows the in-situ infrared spectra of pyrrole adsorption of Catalyst E as a function of time.

## DETAILED DESCRIPTION OF INVENTION

The specific implementation of the present application is described in detail below in conjunction with the accompanying drawings. It should be understood that the specific implementation described here is only used to illustrate and explain the present application, and is not used to limit the present application.

The term "exemplary" is used exclusively herein to mean "serving as an example, embodiment, or illustration." Any example described herein as "exemplary" is not necessarily to be construed as preferred or advantageous over other examples. Although various aspects of the examples are shown in the drawings, the drawings are not necessarily drawn to scale unless otherwise indicated.

Any specific numerical value disclosed herein (including the endpoint of the numerical range) is not limited to the exact value of the numerical value, but should be understood to also encompass values close to the exact value, such as all possible values within the range of ±5% of the exact value. In addition, for the disclosed numerical range, two of the endpoint values of the range, one of the endpoint values and one of the specific point values in the range, and two of the specific point values can be arbitrarily combined to obtain one or more new numerical ranges, and these new numerical ranges should also be regarded as specifically disclosed herein.

Unless otherwise specified, the terms used herein have the same meaning as commonly understood by those skilled in the art. If a term is defined herein and its definition is different from the commonly understood meaning in the art, the definition herein shall prevail.

In the present application, the term "polyunsaturated hydrocarbon" refers to an aliphatic hydrocarbon compound having two or more (e.g., 2-4) unsaturated bonds selected from carbon-carbon double bonds and carbon-carbon triple bonds, such as dienes having 3-5 carbon atoms.

In the present application, except for the contents clearly stated, any matters or items not mentioned shall directly follow those known in the art without any changes. Moreover, any embodiment described herein can be freely combined with one or more other embodiments described herein, and the technical solutions or technical ideas formed thereby are deemed to be part of the original disclosure or original record of this application, and should not be regarded as new contents not disclosed or anticipated herein, unless those skilled in the art consider that the combination is obviously unreasonable.

All patent and non-patent literatures, including but not limited to textbooks and journal articles, mentioned herein are incorporated by reference in their entirety.

The inventors of the present application have discovered through extensive research that, for a supported palladium hydrogenation catalyst, the introduction of weakly basic sites into an overall weakly acidic surface environment of the catalyst can significantly improve the performance of the catalyst, such as activity, selectivity and/or operating life, thereby completing the present invention.

As described above, the present application provides in a first aspect a supported palladium catalyst, comprising a support, and palladium and a modifying component supported on the support, wherein according to an analysis on in-situ infrared spectroscopy of pyrrole adsorption carried out at 40°C, the catalyst exhibits absorption peak(s) in the range of 3251-3410 cm$^{-1}$, preferably in the range of 3350-3400 cm$^{-1}$.

According to the present application, in specific embodiments, the in-situ infrared spectrum of pyrrole adsorption of the catalyst can be measured by the following method:

a) placing the powdered catalyst sample in an infrared cell, subjecting it to vacuum treatment, and then heating to 350°C at a heating rate of 20°C/min;
b) maintaining at 350°C under vacuum state for 2 h, then cooling to 40°C at a cooling rate of 20°C/min;
c) maintaining the temperature at 40°C, and introducing nitrogen to purge for 30 minutes at a nitrogen flow rate of 5 mL/min;
d) maintaining the temperature at 40°C, introducing gaseous pyrrole for adsorption over 10 minutes at a gas flow rate of 5 mL/min, and recording the in-situ infrared spectrum of the sample;
e) maintaining the temperature at 40°C, switching to nitrogen to purge for 30 minutes at a nitrogen flow rate of 5 mL/min, and recording the in-situ infrared spectrum of the sample.

After the measurement, nitrogen purging can be maintained until the temperature drops to room temperature, with a nitrogen flow rate of 3-7 mL/min.

In the above specific embodiments, prior to testing, the granular catalyst sample is first ground into powder, filled into the in-situ sample cell and leveled. Steps a) to c) aim to remove water and impurities adsorbed on the catalyst surface. In the pyrrole adsorption step d), the in-situ infrared spectrum of the sample may also be recorded at different times as needed. For example, the in-situ infrared spectrum of the sample may be recorded at 1 minute, 2 minutes, 3 minutes, 4 minutes, 5 minutes, 6 minutes, 7 minutes, 8 minutes, 9 minutes, and 10 minutes, respectively.

In the in-situ infrared spectrum of pyrrole adsorption of solid catalyst, N-H stretching vibration absorption peak(s) appear in the range of 3150-3410 cm$^{-1}$. The position of the absorption peak(s) characterizes the basic strength of the basic site on the surface of the catalyst. The higher the wave number of the peak position (i.e., the closer to 3410 cm$^{-1}$), the weaker the basic strength of the basic site (see "Recent Advances in Studying Catalyst Basicity by Infrared Spectroscopy", Journal of Taiyuan University of Technology, Vol. 24, pp. 101-107, September 1993). If multiple N-H stretching vibration absorption peaks appear in the range of 3150-3410 cm$^{-1}$, it indicates diversified basic sites on the surface of the catalyst. The peak positions of the in-situ infrared spectra of pyrrole adsorption vary significantly among different solid catalysts. When multiple absorption peaks appear, two or more peaks overlap, and in some cases one peak may appear as a shoulder of another peak. In order to calculate the peak positions of each absorption peak, common data processing software may be used for peak fitting of the absorption curve in the range of 3150-3410 cm$^{-1}$.

In the in-situ infrared spectrum of pyrrole adsorption measured at 40°C of the supported palladium catalyst according to the present application, N-H stretching vibration absorption peak(s) is shown in the range of 3251-3410 cm$^{-1}$, for example, at 3260 cm$^{-1}$, 3270 cm$^{-1}$, 3300 cm$^{-1}$, 3320 cm$^{-1}$, 3340 cm$^{-1}$, 3360 cm$^{-1}$, 3380 cm$^{-1}$, 3400 cm$^{-1}$, 3410 cm$^{-1}$ and any position within the range consisting of any two values therein, preferably in the range of 3350-3400 cm$^{-1}$, indicating the presence of extremely weak basic sites on the surface of the catalyst.

Without being bound by any specific theory, the inventors of the present application have found that when the supported palladium catalyst features an overall weakly acidic surface and is used for hydrogenation of unsaturated hydrocarbon (especially selective hydrogenation of alkyne and polyunsaturated hydrocarbon), it facilitates the proceeding of the hydrogenation reaction, more effectively than a catalyst with a basic surface. Furthermore, when the catalyst surface additionally possesses weakly basic sites as described in the present application (corresponding to absorption peak(s) shown in the range of 3251-3410 cm$^{-1}$ of the in-situ infrared spectrum of pyrrole adsorption), the synergistic cooperation between the weakly basic sites on the catalyst surface and the overall weakly acidic environment enables to more effectively inhibit the hydrogenation of mono-olefins to alkanes, and reduce or eliminate the polymerization of alkynes and dienes on the catalyst surface, thereby further improving the catalytic performance of the catalyst, such as the catalytic performance (e.g., catalytic activity and selectivity) in the selective hydrogenation of alkyne and polyunsaturated hydrocarbon (e.g., feedstocks containing propyne (MA) and propadiene (PD)).

During the above-mentioned analysis on in-situ infrared spectrum of pyrrole adsorption, after nitrogen is used for purging in step e), the intensity of the infrared absorption peak(s) in the obtained spectrum will gradually weaken. If the peak height of the absorption peak in the range of 3150-3420 $cm^{-1}$ in the spectrum measured when purging with nitrogen is carried out for 15 minutes changes significantly relative to the peak height of the corresponding absorption peak in the range of 3150-3420 $cm^{-1}$ in the spectrum measured when purging is carried out for 0 minutes, it indicates that there are a significant number of basic sites that have adsorption capacity for pyrrole on the catalyst surface.

In preferred embodiments, according to the analysis on in-situ infrared spectroscopy of pyrrole adsorption carried out at 40°C, the ratio of the peak height of the absorption peak in the range of 3251-3410 $cm^{-1}$ in the in-situ infrared spectrum of the catalyst of the present application measured when purging with nitrogen is carried out for 0 minute after 10 minutes of pyrrole adsorption to the peak height of the corresponding absorption peak in the range of 3251-3410 $cm^{-1}$ in the in-situ infrared spectrum measured when purging with nitrogen is carried out for 15 minutes is more than or equal to 5:1, preferably more than or equal to 10:1, for example, 10:1 to 200:1.

According to the present application, the supported palladium catalyst may or may not exhibit absorption peak(s) in the range of 3160-3250 $cm^{-1}$ in the in-situ infrared spectrum of pyrrole adsorption measured at 40°C. Preferably, the catalyst exhibits no absorption peak in the range of 3200-3250 $cm^{-1}$, more preferably in the range of 3160-3250 $cm^{-1}$, indicating that there are no other stronger basic sites on the surface of the catalyst except for the extremely weak basic site.

In preferred embodiments, according to an analysis on in-situ infrared spectroscopy of pyridine temperature-programmed adsorption-desorption, the difference between the surface adsorbed pyridine concentration measured after desorption at 150°C and the surface adsorbed pyridine concentration measured after desorption at 300°C of the catalyst of the present application is in the range of 0.1-0.25 mmol/g, preferably in the range of 0.1-0.2 mmol/g, and the surface adsorbed pyridine concentration measured after desorption at 300°C is in the range of 0-0.1 mmol/g, preferably in the range of 0-0.08 mmol/g.

In preferred embodiments, according to the analysis on in-situ infrared spectroscopy of pyridine temperature-programmed adsorption-desorption, the ratio of the difference between the surface adsorbed pyridine concentration measured after desorption at 150°C and the surface adsorbed pyridine concentration measured after desorption at 300°C to the surface adsorbed pyridine concentration measured after desorption at 150°C of the catalyst of the present application is in the range of 60-100%, preferably in the range of 75-100%.

According to the present application, in the analysis on in-situ infrared spectroscopy of pyridine temperature-programmed adsorption-desorption of the catalyst, the surface adsorbed pyridine concentration measured after desorption at 150°C of the catalyst characterizes the total amount of acids on the catalyst surface (i.e., the total amount of weak acid, medium-strong acid, and strong acid), the surface adsorbed pyridine concentration of the catalyst measured after desorption at 300°C characterizes the total amount of medium-strong acid and strong acid on the catalyst surface, and the difference between the two characterizes the weak acid content on the catalyst surface. Accordingly, the ratio of the difference between the surface adsorbed pyridine concentration measured after desorption at 150°C and the surface adsorbed pyridine concentration measured after desorption at 300°C to the surface adsorbed pyridine concentration measured after desorption at 150°C of the catalyst characterizes the proportion of the weak acid amount on the catalyst surface relative to the total acid amount (also referred to as the weak acid proportion herein). The acidic sites on the surface of the catalyst include Brønsted acid (B acid for short) and Lewis acid (L acid for short). In the pyridine temperature-programmed adsorption-desorption in-situ infrared spectrum of the catalyst, the peak near the wave number of 1540 $cm^{-1}$ corresponds to B acid, while the peak near the wave number of 1440-1450 $cm^{-1}$ corresponds to L acid.

According to the present application, in the supported palladium catalyst, palladium is used as a catalytically active component, and the content thereof may vary within a relatively wide range. Preferably, relative to the weight of the support and on metal element basis, the content of palladium in the catalyst is 0.01-20 wt%, for example, 0.01 wt%, 0.02 wt%, 0.05 wt%, 0.1 wt%, 0.5 wt%, 1 wt%, 5 wt%, 10 wt%, 20 wt%, and any value within the range formed by any two of above-mentioned values, preferably 0.01-5 wt%, more preferably 0.02-1 wt%.

According to the present application, palladium may exist on the catalyst surface in an oxidized state, an elemental state, other palladium compound states, or a hybrid state of two or more thereof.

According to the present application, the support of the supported palladium catalyst may be a support conventionally used in the art. For example, the support may be selected from refractory metal oxides, silicon oxide, activated carbon, or combinations thereof, preferably selected from aluminum oxide, zirconium oxide, gallium oxide, silicon oxide, activated carbon, or combinations thereof.

According to the present application, there is no strict requirement for the shape of the catalyst. For example, it may include but is not limited to powders, granules, spheres, toothed spheres, Raschig rings, strips, cylinders, flakes or trilobes, etc. Preferably, the specific surface area of the catalyst is 0.5-800 $m^2$/g, more preferably 4-200 $m^2$/g, and further preferably 15-110 $m^2$/g.

According to the present application, the supported palladium catalyst further comprises a modifying component, and the modifying component may be a modifying component conventionally used in the art. For example, it may be selected from Bi, Sb, Pb, Sn, Zn, W, Mn, Si, Re, Group VIII elements other than palladium (for example, Fe, Co, Ni, Ru, Rh, Os, Ir, Pt), alkali metal elements (for example, Na, K), alkaline earth metal elements (for example, Mg, Ca, Sr, Ba), Group IIIA elements (for example, Ga, In, Tl), Group IB elements (for example, Cu, Ag, Au), rare earth elements (for example, La, Ce, Pr, Nd, Pm, Sm, Eu, Gd, Tb, Dy, Ho, Er, Yb, Lu, Y), halogen elements (for example, F, Cl, Br, I), or combinations thereof, preferably selected from La, K, Ag, Zn, Ga, Cu, Au, Cs, Bi, Mg, Fe, Re, or combinations thereof.

According to the present application, the modified component may be supported on the support together with palladium as a co-active component, or may be used as a support modifier and uniformly distributed in the support. In preferred embodiments, relative to the weight of the support and on metal element basis, the content of the modified component in the catalyst is 0.01-20 wt%, for example, 0.01 wt%, 0.05 wt%, 0.1 wt%, 0.5 wt%, 1 wt%, 5 wt%, 10 wt%, 15 wt%, 20 wt%, and any value within the range formed by any two of above-mentioned values, preferably 0.2-5 wt%.

In a second aspect, provided is a method for preparing the supported palladium catalyst of the present application, comprising the following steps:

1) loading palladium and a modifying component onto a support, followed by drying and calcining to obtain an intermediate; and
2) subjecting the intermediate to an alkalization treatment with an alkaline solution having a pH value of 7.2-10, preferably 7.5-9, followed by drying and optionally calcining to obtain the catalyst,

wherein the alkalization treatment is achieved by spraying the alkaline solution, the spraying time is 2-8 min, preferably 3-4 min, and the spraying temperature is 35-70°C, preferably 45-55°C.

In preferred embodiments, the temperature fluctuation during the spraying process is controlled within 5°C; that is, the difference between the maximum spraying temperature and the minimum spraying temperature during the spraying process is maintained within the range of 0-5°C. More preferably, the spraying is constant temperature spraying.

In the second aspect of the present application, the specific selection of the modifying component and the support is as described in the first aspect of the present application, and will not be reiterated here.

In the method of the present application, there is no strict requirement for the shape of the support, for example, it may include but is not limited to powders, granules, spheres, toothed spheres, Raschig rings, strips, cylinders, flakes or trilobes, etc. Preferably, the specific surface area of the support is 0.5-800 $m^2$/g, more preferably 4-200 $m^2$/g, and further preferably 15-110 $m^2$/g.

In some specific embodiments, the loading of the palladium and the modifying component may be carried out by using a solution containing a precursor of palladium and a solution containing a precursor of the modifying component, respectively, or by using a mixed solution containing a precursor of palladium and a precursor of the modifying component. The specific method for carrying out the loading may be a method conventionally used in the art, such as a spraying method or an impregnation method, preferably a spraying method and an incipient wetness impregnation method, and the specific operation will not be reiterated.

According to the present application, the precursor of palladium may be a soluble palladium compound conventionally known in the art. For example, it may be selected from palladium chloride, palladium nitrate, palladium acetate, palladium sulfate, palladium compounds containing organic group(s) (such as palladium pivalate, palladium octaethylporphyrin, palladium trimethylacetate, palladium trifluoroacetate, etc.), or combinations thereof, preferably selected from palladium chloride, palladium nitrate, palladium acetate, palladium sulfate, or combinations thereof.

In preferred embodiments, the solution containing the precursor of palladium used in step 1) has a pH value of 2-5, preferably 3.8-4.5.

According to the present application, the content of the precursor of palladium in the solution containing the precursor of palladium may vary within a relatively wide range; for example, the content of the precursor of palladium in the solution may be 1-200 g/L. Preferably, relative to the weight of the support and on metal element basis, palladium is used in such an amount that the content of palladium in the resulting catalyst is 0.01-20 wt%, more preferably 0.01-5 wt%, and further preferably 0.02-1 wt%. In other words, on metal element basis, relative to 100 parts by weight of the support, palladium is used in an amount of 0.01-20 parts by weight, more preferably 0.01-5 parts by weight, and further preferably 0.02-1 parts by weight.

According to the present application, the precursor of the modified component may be a soluble compound containing the modified component conventionally known in the art. For example, it may be selected from halides (such as chlorides, bromides, iodides), nitrates, acetates, carbonates, sulfates, hydroxides, amides, compounds containing organic group(s) (such as citrates, oxalates, etc.) of the modified component, and combinations thereof. Those skilled in the art can determine the corresponding precursor compound according to the specific modified component selected, and will not be listed exhaustively here.

According to the present application, the content of the precursor of the modified component in the solution containing the precursor of the modified component may vary within a relatively wide range; for example, the content of the precursor of the modified component in the solution may be 0.1-400 g/L. Preferably, relative to the weight of the support and on metal element basis, the modified component is used in such an amount that the content of the modified component in the resulting catalyst is 0.01-20 wt%, for example, 0.01 wt%, 0.05 wt%, 0.1 wt%, 0.5 wt%, 1 wt%, 5 wt%, 10 wt%, 20 wt%, and any value within the range formed by any two of above-mentioned values, preferably 0.2-5 wt%. In other words, on metal element basis, relative to 100 parts by weight of the support, the modified component is used in an amount of 0.01-20 parts by weight, preferably 0.2-5 parts by weight.

According to the present application, the solvents in the solution of the precursor containing palladium and the solution of the precursor containing the modifying component may be each independently selected from water, diethyl ether, ethanol, isobutanol and the like.

In preferred embodiments, in step 1), the drying is carried out at a temperature of 50-220°C, preferably 80-150°C, for a time of 1-48 h, preferably 2-24 h; the calcining is carried out at a temperature of 300-1500°C, preferably 350-1250°C, for a time of 2-24 h, preferably 6-24 h, under atmosphere selected from an inert gas, nitrogen, an oxygen-containing atmosphere, or combinations thereof, wherein the atmosphere for calcining has an oxygen content of 0-40 wt%.

According to the present application, in step 1), the modifying component may be loaded onto the support together with palladium or separately. For example, palladium and the modifying component may be loaded onto the support together, or the modifying component may be loaded on the support first, followed by palladium and optional additional modifier component(s).

In certain preferred embodiments, in the step 1), palladium and the modifying component are loaded onto the support together.

In such preferred embodiments, in step 1), the drying may be carried out at a temperature of 60-180°C, for example, 60°C, 80°C, 120°C, 160°C, 180°C, and any value within the range formed by any two of above-mentioned values, preferably 80-150°C. The drying may be each independently carried out for a time of 1-48 h, for example, 1 h, 5 h, 10 h, 20 h, 30 h, 40 h, 48 h, and any value within the range formed by any two of above-mentioned values, preferably 2-24 h. The drying may be carried out under an inert atmosphere, a nitrogen atmosphere, an air atmosphere or vacuum, preferably under an air atmosphere. The drying may be carried out once or multiple times, and the drying conditions may be different each time.

In such preferred embodiments, in the step 1), the calcining may be carried out at a temperature of 300-700°C, for example, 300°C, 400°C, 500°C, 600°C, 700°C, and any value within the range formed by any two of above-mentioned values, preferably 350-600°C, for example, 350°C, 400°C, 500°C, 600°C, and any value within the range formed by any two of above-mentioned values. The calcining may be carried out for a time of 2-24 h, for example, 2 h, 4 h, 8 h, 12 h, 16 h, 20 h, 24 h, and any value within the range formed by any two of above-mentioned values, preferably 6-15 h. The calcining is carried out under an atmosphere selected from an inert gas, nitrogen, an oxygen-containing atmosphere, or combinations thereof, preferably selected from nitrogen, air, or a mixed atmosphere of an inert gas and oxygen. Further preferably, the atmosphere for calcining has an oxygen content of 0-40 wt%, for example, 0 wt%, 5 wt%, 10 wt%, 20 wt%, 30 wt%, 40 wt%, and any value within the range formed by any two of above-mentioned values.

In some other preferred embodiments, the step 1) further comprises:

1a) loading the modified component onto the support, followed by drying and calcining to obtain a modified support; and
1b) loading palladium and optionally an additional modifying component onto the support, followed by drying and calcining to obtain the intermediate.

In such preferred embodiments, when the additional modifying component is used in the step 1b), it may be the same as or different from the modifying component used in the step 1a). The specific selection is as described in the first aspect of the present application and will not be reiterated here.

In further preferred embodiments, in the step 1a), the drying may be carried out at a temperature of 50-220°C, for example, 50°C, 60°C, 80°C, 120°C, 160°C, 180°C, 200°C, 210°C, 220°C, and any value within the range formed by any two of above-mentioned values, preferably 80-150°C. The drying may be carried out for a time of 1-48 h, for example, 1 h, 5 h, 10 h, 20 h, 30 h, 40 h, 48 h, and any value within the range formed by any two of above-mentioned values, preferably 2-12 h. The drying may be carried out under an inert atmosphere, a nitrogen atmosphere, an air atmosphere or vacuum, preferably under an air atmosphere. The drying may be carried out once or multiple times, and the drying conditions may be different each time.

In further preferred embodiments, in the step 1a), the calcining may be carried out at a temperature of 700-1500°C, for example, 700°C, 800°C, 1000°C, 1200°C, 1400°C, 1500°C, and any value within the range formed by any two of above-mentioned values; preferably 800-1250°C, for example, 800°C, 900°C, 1000°C, 1100°C, 1200°C, 1250°C, and any value within the range formed by any two of above-mentioned values. The calcining may be carried out for a time of 2-24 h, for example, 2 h, 4 h, 8 h, 12 h, 16 h, 20 h, 24 h, and any value within the range formed by any two of above-mentioned values, preferably 6-15 h. The calcining is carried out under an atmosphere selected from an inert gas, nitrogen, an oxygen-containing atmosphere, or combinations thereof, preferably selected from nitrogen, air, or a mixed atmosphere of an inert gas and oxygen. Further preferably, the atmosphere for calcining has an oxygen content of 0-40 wt%, for example, 0 wt%, 5 wt%, 10 wt%, 20 wt%, 30 wt%, 40 wt%, and any value within the range formed by any two of above-mentioned values, and air atmosphere is particularly preferred.

In further preferred embodiments, in the step 1b), the drying may be carried out at a temperature of 60-180°C, for example, 60°C, 80°C, 120°C, 160°C, 180°C, and any value within the range formed by any two of above-mentioned values, preferably 80-150°C. The drying may be each independently carried out for a time of 1-48 h, for example, 1 h, 5 h, 10 h, 20 h, 30 h, 40 h, 48 h, and any value within the range formed by any two of above-mentioned values, preferably 2-24 h. The drying may be carried out under an inert atmosphere, a nitrogen atmosphere, an air atmosphere or vacuum, preferably under an air atmosphere. The drying may be carried out once or multiple times, and the drying conditions may be different each time.

In further preferred embodiments, in the step 1b), the calcining may be carried out at a temperature of 300-700°C, for example, 300°C, 400°C, 500°C, 600°C, 700°C, and any value within the range formed by any two of above-mentioned values, preferably 350-650°C, for example, 350°C, 400°C, 500°C, 600°C, 650°C, and any value within the range formed by any two of above-mentioned values. The calcining may be carried out for a time of 2-24 h, for example, 2 h, 4 h, 8 h, 12 h, 16 h, 20 h, 24 h, and any value within the range formed by any two of above-mentioned values, preferably 6-15 h. The calcining is carried out under an atmosphere selected from an inert gas, nitrogen, an oxygen-containing atmosphere, or combinations thereof, preferably selected from nitrogen, air, or a mixed atmosphere of an inert gas and oxygen. Further preferably, the atmosphere for calcining has an oxygen content of 0-40 wt%, for example, 0 wt%, 5 wt%, 10 wt%, 20 wt%, 30 wt%, 40 wt%, and any value within the range formed by any two of above-mentioned values.

According to the present application, the alkaline compound contained in the alkaline solution used in the step 2) is not strictly limited, as long as it can provide an alkaline solution with a desired pH value, for example, it may be an inorganic alkaline compound, an organic alkaline compound or mixtures thereof, preferably a weakly alkaline compound.

In preferred embodiments, the organic alkaline compound is selected from organic amines (such as aliphatic amines, alcohol amines, amides, aromatic amines), pyridine compounds, or combinations thereof, more preferably selected from dimethylamine, triethylamine, trifluoroacetamide, pyridine, 4-dimethylaminopyridine, triethanolamine, or combinations thereof.

In preferred embodiments, the inorganic alkaline compound is selected from ammonia water, halides (such on K basisF, NaF, lithium chloride), citrates (such as sodium citrate or potassium citrate), oxalates (such as sodium oxalate, potassium oxalate), acetates (such as sodium acetate, potassium acetate), bicarbonates (such as sodium bicarbonate, potassium bicarbonate), carbonates (such as sodium carbonate, potassium carbonate), or combinations thereof, preferably selected from KF, lithium chloride, potassium acetate, sodium bicarbonate, sodium citrate, or combinations thereof.

According to the present application, the solvent in the alkaline solution may be any solvent that can dissolve the above-mentioned alkaline compound, preferably selected from water, ethanol, acetone, tetrahydrofuran, or combinations thereof.

In preferred embodiments, the concentration of the alkaline compound in the alkaline solution is 0.1-70 wt%, for example, 0.1 wt%, 0.5 wt%, 1 wt%, 5 wt%, 10 wt%, 20 wt%, 30 wt%, 40 wt%, 70 wt% and any value within the range formed by any two of above-mentioned values.

In certain preferred embodiments, relative to the weight of the support, the alkaline compound in the step 2) is used in an amount of 0.1-25 wt%, for example, 0.1 wt%, 0.5 wt%, 1 wt%, 2 wt%, 4 wt%, 6 wt%, 8 wt%, 10 wt%, 15 wt%, 20 wt%, 25 wt%, and any value within the range formed by any two of above-mentioned values. In other words, relative to 100 parts by weight of the support, the alkaline compound is used in an amount of 0.1-25 parts by weight.

In preferred embodiments, in the step 2), the drying is carried out at a temperature of 60-180°C, for example, 60°C, 80°C, 120°C, 160°C, 180°C, and any value within the range formed by any two of above-mentioned values, preferably 70-140°C. The drying is carried out for a time of 1-48 h, for example, 1 h, 5 h, 10 h, 20 h, 30 h, 40 h, 48 h, and any value within the range formed by any two of above-mentioned values, preferably 6-20 h. The drying may be carried out under an inert atmosphere, a nitrogen atmosphere, an air atmosphere or vacuum, preferably under an air atmosphere. The drying may be carried out once or multiple times, and the drying conditions may be different each time.

In preferred embodiments, in the step 2), the calcining is carried out at a temperature of 200-500°C, for example, 200°C, 250°C, 300°C, 350°C, 400°C, 450°C, 500°C, and any value within the range formed by any two of above-mentioned values, preferably 250-450°C, for example, 250°C, 300°C, 400°C, 450°C, and any value within the range formed by any two of above-mentioned values. The calcining may be carried out for a time of 2-24 h, for example, 2 h, 4 h, 8 h, 12 h, 16 h, 20 h, 24 h, and any value within the range formed by any two of above-mentioned values, preferably 6-20 h. The calcining is carried out under an atmosphere selected from an inert gas, nitrogen, an oxygen-containing atmosphere, or combinations thereof, preferably selected from nitrogen, air, or a mixed atmosphere of an inert gas and oxygen. Further preferably, the atmosphere for calcining has an oxygen content of 0-25 wt%, for example, 0 wt%, 5 wt%, 10 wt%, 15 wt%, 20 wt%, 25 wt%, and any value within the range formed by any two of above-mentioned values.

The method for preparing the catalyst of the present application provides an overall weakly acidic surface environment and specific weakly basic sites for the catalyst by loading a modified component on the catalyst, in combination with a specific alkalization treatment step, so that the catalyst exhibits better catalytic activity and selectivity in the hydrogenation of unsaturated hydrocarbon (especially the selective hydrogenation of alkyne and polyunsaturated hydrocarbon, such as feedstocks containing propyne and propadiene (MAPD)). Moreover, the preparation method is simple and easy to implement, facilitating industrial production.

In a third aspect, provided is use of the supported palladium catalyst of the present application in hydrogenation of unsaturated hydrocarbon, especially in selective hydrogenation of alkyne and polyunsaturated hydrocarbon.

In a fourth aspect, provided is a method for hydrogenating unsaturated hydrocarbon, comprising a step of contacting an unsaturated hydrocarbon feedstock comprising alkyne and/or polyunsaturated hydrocarbon with the supported palladium catalyst of the present application in the presence of hydrogen for reaction.

The catalyst of the present application is suitable for the hydrogenation of various alkyne and polyunsaturated hydrocarbon, wherein the alkyne may be selected from C2-C5 alkynes, preferably selected from C2-C3 alkynes, and the polyunsaturated hydrocarbon may be selected from C3-C5 dienes, preferably selected from C3-C4 dienes.

Feedstocks suitable for selective hydrogenation using the supported palladium catalyst of the present application include various feedstocks containing dienes and/or alkynes commonly found in the chemical industry, such as feedstocks containing dienes and/or alkynes in catalytic cracking or steam cracking processes, such as C2, C3 or C4 fractions, and in particular feedstocks containing MAPD.

Those skilled in the art can select appropriate operating conditions to achieve the reaction according to the specific reaction type. For example, in certain specific embodiments, the method includes contacting a C3 fraction containing MAPD with the supported palladium catalyst of the present application in the presence of hydrogen under the conditions of an inlet temperature of 20-60°C, a molar ratio of hydrogen to the sum of propyne (MA) and propadiene (PD) of 1.0-5.0:1, and a reaction pressure of 1.0-4.0 MPa to selectively hydrogenate MAPD in the fraction into propylene in liquid phase.

In other specific embodiments, the method comprises contacting a cracked gas stream containing C2 fraction, C3 fraction, hydrogen, CO, methane, and a small amount of C4 fraction with the supported palladium catalyst of the present application in the presence of hydrogen under the conditions of an inlet temperature of 20-120°C, a reaction pressure of 1.0-4.0 MPa, and a gas phase space velocity of 5000-30000 h$^{-1}$ to selectively hydrogenate acetylene and part of MAPD in the cracked gas stream into ethylene and propylene.

## Examples

The present application will be further described below in conjunction with examples, but the scope of the present application is not limited to these examples.

Unless otherwise specified, in the following examples and comparative examples, the reagents used are all reagents conventionally used in the art, and the methods adopted are all conventional methods in the art.

The Al$_2$O$_3$ support used in the following examples and comparative examples was all purchased from Sinopec Catalyst (Beijing) Co., Ltd., with a specific surface area of 10-220 m$^2$/g; the Al$_2$O$_3$-Ga$_2$O$_3$ mixed support was from Sinopec (Beijing) Chemical Research Institute Co., Ltd., with a specific surface area of 10-200 m$^2$/g; the activated carbon support was from Sinopec (Beijing) Chemical Research Institute Co., Ltd., prepared with organic polymer as the base material, with a specific surface area of 100-400 m$^2$/g.

In the following examples and comparative examples, the specific composition of the obtained catalyst can be calculated based on the amount of the support, Pd and the modified component, and the reagents used for alkalization treatment and adjusting the pH value of the solution were not included in the catalyst composition.

In the following examples and comparative examples, unless otherwise indicated, the calcination atmosphere used was air.

Example 1

250 mL of an aqueous solution containing Pd(NO$_3$)$_2$ (0.3 g on Pd basis), AgNO$_3$ (0.5 g on Ag basis), and Zn(NO$_3$)$_2$ (1.1 g on Zn basis) was prepared. Ammonia water was introduced into the solution to adjust the pH to 4.1. 500 g of spherical support Al$_2$O$_3$ (with a specific surface area of 112 m$^2$/g) was impregnated in the solution in equal volumes, dried at 105°C for 12 h, and calcined at 650°C for 15 h to obtain a Pd-Zn-Ag/Al$_2$O$_3$ intermediate.

The amount of KF added was adjusted to prepare 250 mL of an aqueous solution of KF with a pH of 7.5. The solution was sprayed on the Pd-Zn-Ag/Al$_2$O$_3$ intermediate at a constant temperature of 45°C for a spraying time of 4 min, dried at 160°C for 4 h, and calcined at 300°C for 4 h to obtain catalyst A with a specific surface area of 101 m$^2$/g.

Example 2

400 mL of an ethanol solution of copper acetate containing 3.1 g Cu was prepared. 500 g of toothed spherical

support $Al_2O_3$ (with a specific surface area of 201 $m^2/g$) was impregnated in the solution, dried at 75°C for 40 h, and calcined at 1100°C for 20 h to obtain a Cu-modified support $Al_2O_3$.

280 mL of an aqueous solution of $Pd(Cl)_2$ containing 0.5 g Pd was prepared. $(NH_4)_2CO_3$ was added to the solution to adjust the pH to 5.0. The Cu-modified support $Al_2O_3$ was impregnated in the solution, dried at 105°C for 6 h, and calcined at 700°C for 4 h to obtain a Pd-Cu/$Al_2O_3$ intermediate.

The amount of tetramethylethylenediamine added was adjusted to prepare an aqueous solution of tetramethylethylenediamine with a pH of 8.6. 300 mL of the solution was sprayed on the Pd-Cu/$Al_2O_3$ intermediate at a constant temperature of 40°C for a spraying time of 6 min, followed by stepwise drying at 75°C for 2 h and 140°C for 8 h to obtain catalyst B with a specific surface area of 47 $m^2/g$.

Example 3

400 mL of an acetic acid solution containing $Pd(OAc)_2$ (1.2 g on Pd basis) and $Cs(OAc)$ (5.0 g on Cs basis) was prepared. $Na_2CO_3$ was added to the solution to adjust the pH to 3.0. 500 g of trilobate support $Al_2O_3$ (with a specific surface area of 18 $m^2/g$) was impregnated in the solution, dried at 80°C for 48 h, and calcined at 450°C for 8 h to obtain a Pd-Cs/$Al_2O_3$ intermediate.

The amount of $K(OAc)$ added was adjusted to prepare 400 mL of an ethanol solution of $K(OAc)$ with a pH of 8.2. The solution was sprayed on the Pd-Cs/$Al_2O_3$ intermediate at a constant temperature of 60°C for a spraying time of 2 min. The catalyst was dried at 80°C for 3 h and calcined at 400°C for 6 h to obtain catalyst C with a specific surface area of 15 $m^2/g$.

Example 4

265 mL of isobutanol solution of $HAuCl_4$ containing 0.5 g Au was prepared. 500 g of cylindrical support $Al_2O_3$ (with a specific surface area of 53 $m^2/g$) was impregnated in the solution, dried at 110°C for 2 h, and calcined at 1270°C for 6 h to obtain an Au-modified support $Al_2O_3$.

300 mL of an aqueous solution containing $Pd(NO_3)_2$ (4.8 g on Pd basis) and $Ga(NO_3)_3$ (0.8 g on Ga basis) was prepared. $Na_2CO_3$ was added to the solution to adjust the pH to 4.5. The solution was sprayed on the Au-modified support $Al_2O_3$, dried at 140°C for 12 h, and calcined at 470°C for 14 h to obtain a Pd-Ga-Au/$Al_2O_3$ intermediate.

The amount of 4-dimethylaminopyridine (DMAP) added was adjusted to prepare an acetone solution of DMAP with a pH of 9.5. 300 mL of the solution was sprayed on the Pd-Ga-Au/$Al_2O_3$ intermediate at a constant temperature of 55°C for a spraying time of 3 min, followed by stepwise drying at 80°C for 2 h and at 180°C for 12 h to obtain catalyst D with a specific surface area of 27 $m^2/g$.

Example 5

610 mL of an aqueous solution containing $La(NO_3)_3$ (0.5 g on La basis) and $KNO_3$ (1.0 g on K basis) was prepared. 500 g of spherical support $Al_2O_3$ (with a specific surface area of 146 $m^2/g$ ) was impregnated in the solution, dried at 80°C for 12 h, and calcined at 1120°C for 8 h to obtain a La and K modified support $Al_2O_3$.

180 mL of an aqueous solution of $PdSO_4$ containing 2.5 g Pd was prepared. $NaHCO_3$ was gradually added to the solution to adjust the pH to 4.2. The solution was sprayed on the La and K modified support $Al_2O_3$, dried at 140°C for 12 h, and calcined at 620°C for 10 h to obtain a Pd-La-K/$Al_2O_3$ intermediate.

The amount of $NaHCO_3$ added was adjusted to prepare 180 mL of an aqueous solution of $NaHCO_3$ with a pH of 7.8. The solution was sprayed on the Pd-La-K/$Al_2O_3$ intermediate at a constant temperature of 50°C for a spraying time of 3.5 min, dried at 125°C for 10 h, and calcined at 445°C for 16 h to obtain catalyst E with a specific surface area of 56 $m^2/g$.

Comparative Example 1

The amount of $NaHCO_3$ added was adjusted to prepare 450 mL of an aqueous solution of $NaHCO_3$ with a pH of 7.8. 500 g of spherical support $Al_2O_3$ (with a specific surface area of 146 $m^2/g$) was impregnated in the solution at a temperature of 50°C and an impregnation time of 6 min, dried at 125°C for 10 h, and calcined at 1050°C for 20 h to obtain an alkalization treated $Al_2O_3$ intermediate.

180 mL of an aqueous solution of $PdSO_4$ containing 2.5 g Pd was prepared. The solution was sprayed on 500 g of alkalization treated Na/$Al_2O_3$ intermediate, dried at 140°C for 12 h, and calcined at 620°C for 10 h to obtain catalyst F with a specific surface area of 78 $m^2/g$.

Comparative Example 2

220 mL of an aqueous solution of $PdSO_4$ containing 2.5 g Pd was prepared. The solution was sprayed on 500 g of spherical support $Al_2O_3$ (with a specific surface area of 146 $m^2/g$), dried at 140°C for 6 h, and calcined at 620°C for 10 h to obtain catalyst G with a specific surface area of 139 $m^2/g$.

Comparative Example 3

610 mL of an aqueous solution containing $La(NO_3)_3$ (0.5 g on La basis) and $KNO_3$ (1.0 g on K basis) was prepared. 500 g of spherical support $Al_2O_3$ (with a specific surface area of 146 $m^2/g$) was impregnated in the solution, dried at 80°C for 12 h, and calcined at 1120°C for 8 h to obtain a La and K modified support $Al_2O_3$.
180 mL of an aqueous solution of $PdSO_4$ containing 2.5 g Pd was prepared. $NaHCO_3$ was gradually added to the solution to adjust the pH to 4.2. The solution was sprayed on the La and K modified support $Al_2O_3$, dried at 140°C for 6 h, and calcined at 620°C for 10 h to obtain catalyst H with a specific surface area of 62 $m^2/g$.

Comparative Example 4

610 mL of an aqueous solution containing $La(NO_3)_3$ (0.5 g on La basis) and $KNO_3$ (1.0 g on K basis) was prepared. 500 g of spherical support $Al_2O_3$ (with a specific surface area of 146 $m^2/g$) was impregnated in the solution, dried at 80°C for 12 h, and calcined at 1120°C for 8 h to obtain a La and K modified support $Al_2O_3$.
180 mL of an aqueous solution of $PdSO_4$ containing 2.5 g Pd was prepared. $NaHCO_3$ was gradually added to the solution to adjust the pH to 4.2. The solution was sprayed on the La and K modified support $Al_2O_3$, dried at 140°C for 12 h, and calcined at 620°C for 10 h to obtain a $Pd-La-K/Al_2O_3$ intermediate.
The amount of NaOH added was adjusted to prepare 220 mL of an aqueous solution of NaOH with a pH of 12. The solution was sprayed on the $Pd-La-K/Al_2O_3$ intermediate at a constant temperature of room temperature for a spraying time of 5 min, dried at 105°C for 10 h, and calcined at 250°C for 4 h to obtain catalyst I with a specific surface area of 51 $m^2/g$.

Comparative Example 5

610 mL of an aqueous solution containing $La(NO_3)_3$ (0.5 g on La basis) and $KNO_3$ (1.0 g on K basis) was prepared. 500 g of spherical support $Al_2O_3$ (with a specific surface area of 146 $m^2/g$) was impregnated in the solution in equal volumes, dried at 80°C for 12 h, and calcined at 1120°C for 8 h to obtain a La and K modified support $Al_2O_3$.
180 mL of an aqueous solution of $PdSO_4$ containing 2.5 g Pd was prepared. $NaHCO_3$ was gradually added to the solution to adjust the pH to 4.2. The solution was sprayed on the La and K modified support $Al_2O_3$, dried at 140°C for 12 h, and calcined at 620°C for 10 h to obtain a $Pd-La-K/Al_2O_3$ intermediate.
The amount of $NaHCO_3$ added was adjusted to prepare 290 mL of an aqueous solution of $NaHCO_3$ with a pH of 7.8. The $Pd-La-K/Al_2O_3$ intermediate was impregnated in the solution at room temperature for an impregnation time of 25 min, dried at 125°C for 10 h, and calcined at 445°C for 6 h to obtain catalyst J with a specific surface area of 45 $m^2/g$.

Example 6

420 mL of an aqueous solution containing Bi $(NO_3)_3$ (0.2 g on Bi basis) and $MgNO_3$ (2 g on Mg basis) was prepared. 500 g of spherical $Al_2O_3$ -$Ga_2O_3$ mixed support (with a $Ga_2O_3$ content in the support of 20% and a specific surface area of 84 $m^2/g$) was impregnated in the solution, dried at 80°C for 12 h, and calcined at 800°C for 8 h to obtain a Bi and Mg modified $Al_2O_3$-$Ga_2O_3$ mixed support.
320 mL of an aqueous solution of $Pd(Cl)_2$ containing 0.5 g Pd was prepared. $KHCO_3$ was gradually added to the solution to adjust the pH to 4.8. The solution was sprayed on the Bi and Mg modified $Al_2O_3$-$Ga_2O_3$ mixed support, dried at 120°C for 12 h, and calcined at 520°C for 6 h to obtain an intermediate of $Pd-Bi-Mg/Al_2O_3$-$Ga_2O_3$ mixed support.
The amount of lithium chloride added was adjusted to prepare an aqueous solution with a pH of 8.0. 310 mL of the solution was sprayed on the intermediate of $Pd-Bi-Mg/Al_2O_3$-$Ga_2O_3$ mixed support at a constant temperature of 50°C for a spraying time of 5 min, dried at 120°C for 8 h, and calcined at 360°C under a nitrogen atmosphere for 6 h to obtain catalyst K with a specific surface area of 75 $m^2/g$.

Example 7

440 mL of an aqueous solution containing Pd $(NO_3)_2$ (4 g on Pd basis), $Fe(NO_3)_3$ (1 g on Fe basis) and perrhenic acid (0.5 g on Re basis) was prepared. $(NH_4)_2CO_3$ was gradually added to the solution to adjust the pH to 3.5. The solution was sprayed on 500 g of cylindrical support activated carbon (with a specific surface area of 296 $m^2/g$), dried at 105°C for 8 h, and calcined at 360°C under a nitrogen atmosphere for 18 h to obtain an intermediate of Pd-Fe-Re/activated carbon support.

The amount of triethanolamine added was adjusted to prepare an aqueous solution with a pH of 10. 420 mL of the solution was sprayed on the intermediate of Pd-Fe-Re/activated carbon support at a constant temperature of 45°C for a spraying time of 3 min, dried at 105°C for 4 h, and calcined at 270°C under a nitrogen atmosphere for 5 h to obtain catalyst L with a specific surface area of 289 $m^2$/g.

Test Example 1

This test example was used to illustrate the pyrrole in-situ adsorption infrared spectroscopy analysis of the catalysts obtained in the examples and comparative examples.

The catalysts prepared in the examples and comparative examples were tested for pyrrole adsorption using a pyrrole in-situ infrared analyzer (Thermo Nicolet 380). Approximately 10 mg of powdered sample was pressed into a thin sheet, fixed in an infrared cell, vacuum purified (350°C, $1\times10^{-3}$ Pa) for 2 h first, then cooled to 40°C, and scanned to obtain a spectrum as background.

The method for determining the in-situ infrared spectrum of pyrrole adsorption of the catalyst was as follows:

a) placing the powdered catalyst sample in an infrared cell, subjecting it to vacuum treatment, and then heating to 350°C at a heating rate of 20°C/min;

b) maintaining at 350°C under vacuum state for 2 h, then cooling to 40°C at a cooling rate of 20°C/min;

c) maintaining the temperature at 40°C, and introducing nitrogen to purge for 30 minutes at a nitrogen flow rate of 5 mL/min;

d) maintaining the temperature at 40°C, introducing gaseous pyrrole for adsorption over 10 minutes at a gas flow rate of 5 mL/min, and recording the in-situ infrared spectrum of the sample in the wave number region of 1000-4000 $cm^{-1}$ at 2 min, 4 min, 6 min, 8 min, and 10 min after introducing gaseous pyrrole;

e) maintaining the temperature at 40°C, switching to nitrogen to purge for 30 min at a nitrogen flow rate of 5 mL/min; and

f) maintaining nitrogen purging at a nitrogen flow rate of 5 mL/min until the temperature dropped to room temperature.

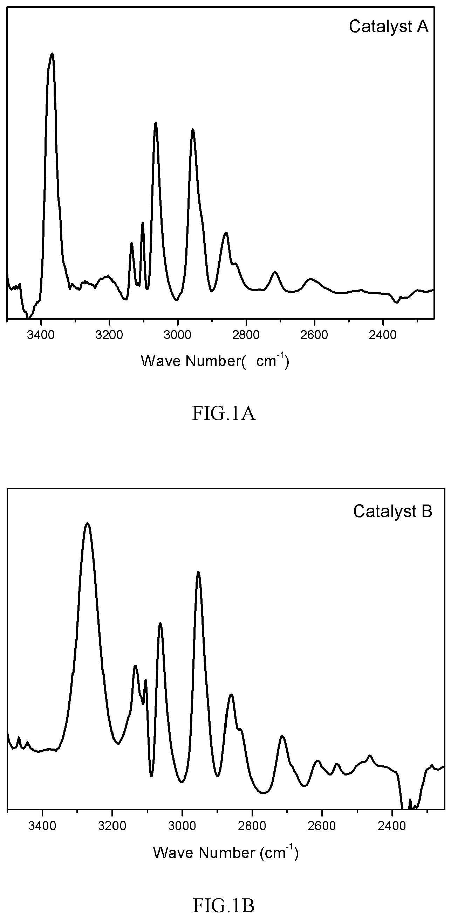

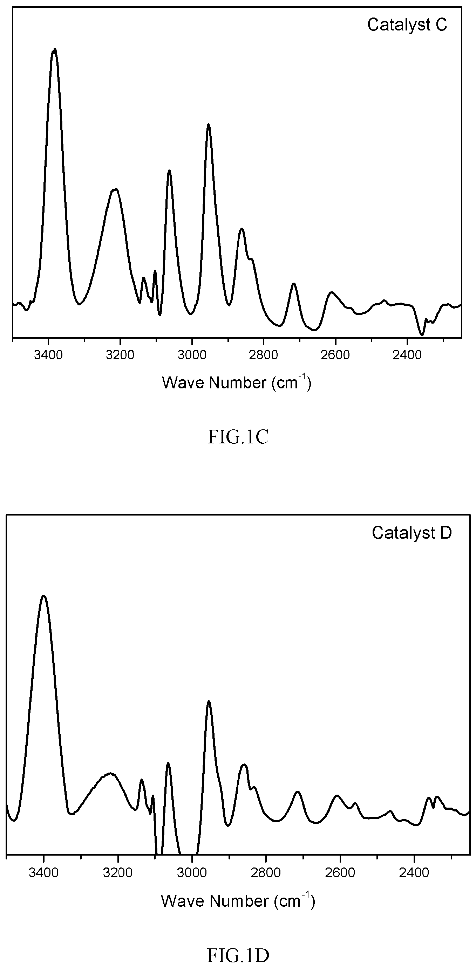

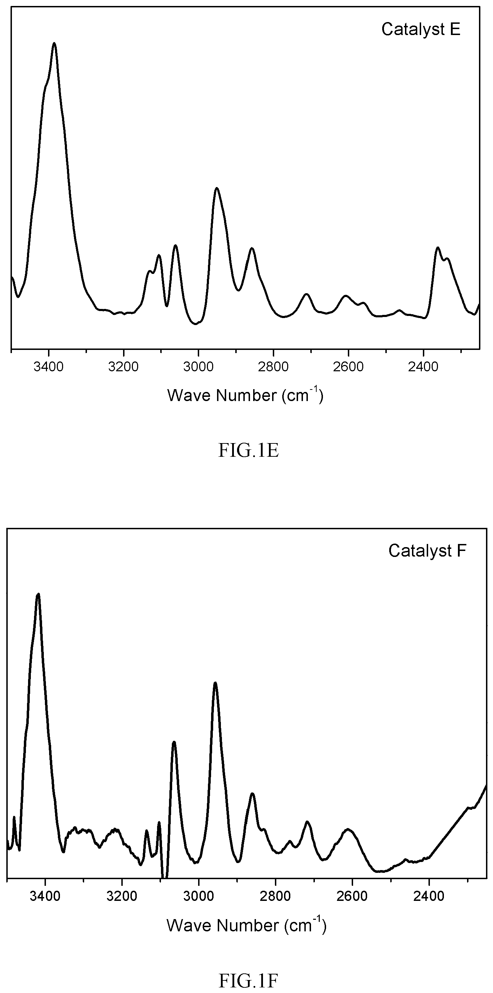

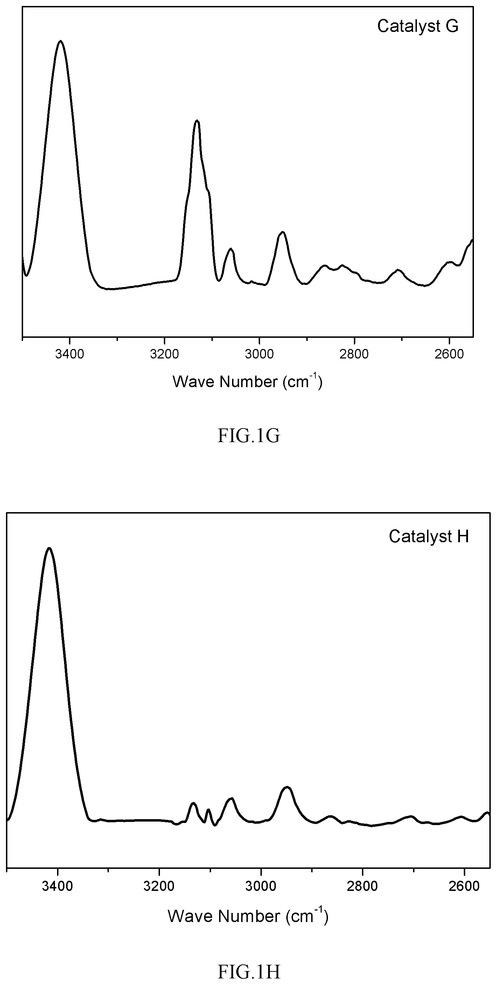

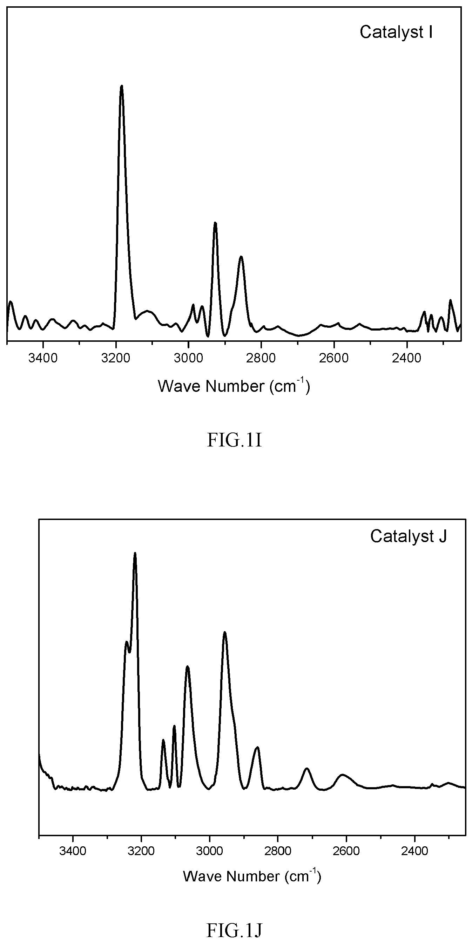

Figures 1A to 1J show the corresponding in-situ infrared spectra of pyrrole adsorption of Catalysts A-J prepared in the examples and comparative examples.

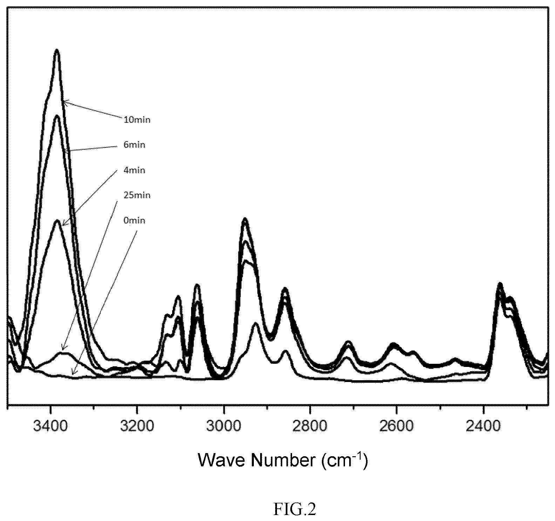

Figure 2 shows the in-situ infrared spectra of pyrrole adsorption of Catalyst E as a function of time. In the spectra, 0 min represents the infrared absorption spectrum at the end of nitrogen purging and before the start of pyrrole adsorption; 4 min, 6 min, and 10 min represent the infrared absorption spectra of pyrrole adsorption for 4 min, 6 min, and 10 min, respectively; and 25 min represents the infrared absorption spectrum when purging with nitrogen again is carried out for 15 min after 10 min of pyrrole adsorption.

Table 1 shows the peak positions of the pyrrole infrared absorption peaks in the range of 3150-3420 $cm^{-1}$ of the catalysts obtained in the examples and comparative examples when pyrrole adsorption is carried out for 10 min, and the peak height ratio $H_{10min}/H_{25min}$, wherein $H_{10min}$ is the peak height of the maximum peak in the range of 3150-3420 $cm^{-1}$ when pyrrole adsorption is carried out for 10 min (i.e., when purging with nitrogen is carried out for 0 min after adsorption for 10 min), and $H_{25min}$ is the peak height of the corresponding peak in the range of 3150-3420 $cm^{-1}$ when purging with nitrogen again is carried out for 15 min after 10 min of pyrrole adsorption.

Table 1. In-situ infrared spectroscopy analysis results of pyrrole adsorption of the catalysts obtained in the examples and comparative examples

| Catalyst No. | Infrared absorption peak after 10 min of pyrrole adsorption | | Peak height ratio $H_{10min}/H_{25min}$ |
|---|---|---|---|
| | Peak position 1 | Peak position 2 | |
| A | 3367.2 | / | 11 |
| B | 3270.7 | / | 37 |
| C | 3382.6 | 3212.9 | 42 |
| D | 3397.9 | 3226.4 | 32 |
| E | 3388.2 | / | 17 |
| F | 3417.3* | / | 54 |
| G | 3419.2* | / | 23 |

(continued)

| Catalyst No. | Infrared absorption peak after 10 min of pyrrole adsorption | | Peak height ratio $H_{10min}/H25min$ |
| --- | --- | --- | --- |
| | Peak position 1 | Peak position 2 | |
| H | 3415.4* | / | 46 |
| I | 3183.9 | / | 42 |
| J | 3244 | 3219 | 39 |
| K | 3325.1 | | 34 |
| L | 3353.7 | 3254.3 | 29 |
| *Infrared absorption peak outside the range of 3150-3410 cm$^{-1}$ does not correspond to basic sites. | | | |

As can be seen from Table 1, the in-situ infrared spectra of pyrrole adsorption of Catalysts A-E and K-L prepared by the method of the examples of the present application all exhibit absorption peak(s) in the wave number range of 3250-3410 cm$^{-1}$; the peak positions of the in-situ infrared spectra of pyrrole adsorption of Catalysts F-H prepared in the comparative examples are greater than 3410 cm$^{-1}$, while the peak positions of the in-situ infrared spectra of pyrrole adsorption of Catalysts I and J are both less than 3250 cm$^{-1}$. After the pyrrole adsorption is completed (i.e., after 10 min of pyrrole adsorption) and nitrogen is used for purging, the pyrrole adsorbed on the catalyst surface will be rapidly desorbed, and the peak height ratio $H_{10min}/H_{25min}$ of each catalyst is above 10. The results of Table 1 indicate that Catalysts A-E and K-L of the present application have special basic sites different from those of Comparative Catalysts F-J.

Test Example 2

This test example is used to illustrate the in-situ infrared spectroscopy analysis of pyridine temperature-programmed adsorption-desorption of the catalysts obtained in the examples and comparative examples.
The catalysts prepared in the examples and comparative examples were tested for temperature-programmed adsorption-desorption using an in-situ infrared analyzer (Thermo Nicolet 380). Approximately 10 mg of powdered sample was pressed into a thin sheet, fixed in an infrared cell, vacuum purified (350°C, 1×10$^{-3}$ Pa) for 2 h first, then cooled to 150°C, and scanned to obtain a spectrum as background. The method for determining the in-situ infrared spectrum of pyridine adsorption of the catalyst was as follows:

a) placing the powdered catalyst sample in an infrared cell, subjecting it to vacuum treatment, and then heating to 350°C at a heating rate of 20°C/min;
b) maintaining at 350°C under vacuum state for 2 h, then cooling to room temperature at a cooling rate of 20°C/min;
c) maintaining room temperature and introducing nitrogen to purge for 30 min at a nitrogen flow rate of 5 mL/min;
d) maintaining room temperature, introducing pyridine for adsorption over 10 min at a gas flow rate of 5 mL/min; and
e) programming the temperature to the measurement temperature (fixed temperature was 150°C and 300°C, respectively) for vacuum desorption (1×10$^{-3}$ Pa) over 0.5 h, and then cooling to room temperature respectively, and recording the infrared spectrum in the wave number region of 1700-1400 cm$^{-1}$, wherein the programmed heating rate was 20°C/min.

The adsorbed pyridine concentration of the sample measured after desorption at 150°C ($C_{150°C}$) represents the total amount of acids of different strengths (including L acid and B acid) on the sample surface, while the adsorbed pyridine concentration of the sample after desorption at 300°C ($C_{300°C}$) represents the total amount of medium-strong acid and strong acid on the sample surface, wherein the absorption peak near 1540 cm$^{-1}$ corresponds to B acid and the absorption peak near 1450 cm$^{-1}$ corresponds to L acid. The adsorbed pyridine concentration of the acidic sites on the catalyst surface at different temperatures was calculated according to the following adsorbed pyridine concentration formula:

$$C_{temperature} = [1.88 * IA(B) + 1.42 * IA(L)] * \frac{R^2}{W}$$

$$C_{weak\ acid} = C_{150°C} - C_{300°C}$$

C represents the concentration of pyridine adsorbed on the acidic sites of the catalyst surface (mmol/g); 1.88 and 1.42 are the extinction coefficients of B acid and L acid, respectively; IA(B) and IA(L) are the integrated peak areas of the characteristic peaks of B acid and L acid, respectively; R represents the radius of the self-supporting molecular sieve tablet (cm); W represents the mass of the tablet (mg).

Table 2 shows the adsorbed pyridine concentrations at the acidic sites on the surfaces of various catalysts measured by in-situ infrared spectroscopy analysis of pyridine temperature-programmed adsorption-desorption.

Table 2. In-situ infrared spectroscopy analysis results of pyridine temperature-programmed adsorption-desorption of the catalysts obtained in the examples and comparative examples

| Catalyst No. | Adsorbed pyridine concentration at the acid sites on the catalyst surface (mmol/g) | | C weak acid | Weak acid percentage (C $_{weak\ acid}$/C$_{150°C}$ ×100%) |
|---|---|---|---|---|
| | C $_{150°C}$ | C $_{300°C}$ | | |
| A | 0.276 | 0.064 | 0.212 | 76.8% |
| B | 0.153 | /* | 0.153 | 100% |
| C | 0.234 | 0.093 | 0.141 | 60.2% |
| D | 0.144 | 0.028 | 0.116 | 80.6% |
| E | 0.170 | / | 0.170 | 100% |
| F | 0.242 | 0.117 | 0.125 | 51.7% |
| G | 0.516 | 0.32 | 0.196 | 38.0% |
| H | 0.192 | / | 0.192 | 100% |
| I | 0.018 | / | 0.018 | 100% |
| J | 0.121 | / | 0.121 | 100% |
| K | 0.185 | 0.021 | 0.164 | 88.6% |
| L | 0.257 | 0.056 | 0.201 | 78.2% |
| *The symbol "/" means not measured. | | | | |

As can be seen from Table 2, the acid amount and acid amount distribution of the catalysts obtained in each examples and comparative examples are different. In terms of the concentration of pyridine adsorbed on the acidic sites, the weak acid amount of Catalysts A-E and K-L obtained in the examples of the present application is in the range of 0.1-0.25 mmol/g, the medium-strong acid and strong acid amount are less than 0.1 mmol/g, the Catalysts H and J obtained in the comparative examples have moderate weak acid amount and no medium-strong and strong acid, the medium-strong and strong acid amount of Catalysts F and G are high, while the acid amount of catalyst I is extremely low, indicating that there is almost no acidic site. The results of Table 2 indicate the overall weakly acidic surfaces of Catalysts A-E and K-L of the present application.

Application Example 1

This example is used to illustrate the effects of the catalysts obtained in the examples and comparative examples on catalytic hydrogenation of C3.

C3 hydrogenation side line was equipped in the ethylene unit, 500 mL of catalyst was charged into an adiabatic fixed-bed reactor, and after nitrogen replacement, a C3 fraction feedstock in liquid phase was introduced into the reactor for catalytic hydrogenation, wherein the composition (molar fraction) of the reaction feedstock was: propane 6.106%, propylene 89.864%, propadiene 1.994%, propyne 2.035%.

The experiment was carried out in the range of 1.0-1.8 molar ratio of hydrogen to propyne and propadiene (MAPD) and 25-55°C inlet temperature of the reactor, and the outlet MAPD content was controlled to be less than 100 ppm. The minimum $H_2$/MAPD ratio and inlet temperature of each catalyst were screened out. The results are shown in Table 3. The catalytic reaction was carried out under the conditions shown in Table 3, at a reaction pressure controlled to be 1.8 MPa, with a liquid phase space velocity of 62 h$^{-1}$. And the selectivity of each catalyst reaction to propylene was calculated. The results are shown in Table 3. The calculation method thereof is:

$$\text{Propylene selectivity} = \frac{(C_3H_6)_{\text{outlet}} - (C_3H_6)_{\text{inlet}}}{(MAPD)_{\text{inlet}} - (MAPD)_{\text{outlet}}} \times 100\%$$

Table 3. C3 hydrogenation test conditions and results of the catalysts obtained in the examples and comparative examples

| Catalyst | Outlet MAPD | Minimum inlet temperature | Minimum $H_2$ /MAPD | Propylene selectivity |
|---|---|---|---|---|
| A | Less than 100 ppm | 33°C | 1.5 | 80% |
| B | Less than 100 ppm | 33°C | 1.5 | 72% |
| C | Less than 100 ppm | 33°C | 1.5 | 74% |
| D | Less than 100 ppm | 28°C | 1.5 | 77% |
| E | Less than 100 ppm | 28°C | 1.5 | 83% |
| F | Less than 100 ppm | 37°C | 1.5 | 39% |
| G | Less than 100 ppm | 39°C | 1.5 | 37% |
| H | Less than 100 ppm | 35°C | 1.7 | 45% |
| I | 1000 ppm* | 43°C | 2.5 | 65% |
| J | 250 ppm* | 43°C | 2.5 | 60% |
| * Catalysts I and J cannot achieve outlet MAPD of less than 100 ppm. | | | | |

As can be seen from Table 3, in the reaction of hydrogenating MAPD into propylene, compared with the catalysts of the comparative examples, Catalysts A-E obtained in the examples of the present application exhibit higher selectivity to propylene while ensuring the outlet MAPD of less than 100 ppm. This demonstrates that the modification of adding extremely weakly basic sites in the weakly acidic environment on the catalyst surface can better improve the catalytic performance of the catalyst, and the catalysts having the specific basic sites of the present application exhibit better catalytic effects.

Application Example 2

This example is used to illustrate the effects of the catalysts obtained in the examples and comparative examples for hydrogenation of a cracked gas stream.

A three-stage adiabatic fixed-bed reactor for tandem hydrogenation was used in the hydrogenation process of cracked gas stream, with a catalyst loading of 200 mL in each stage and a heat exchanger between each stages for controlling the feed temperature. The composition of the cracked gas stream at the reactor inlet was: hydrogen 13.5 mol%, CO 755 ppm, ethylene 33 mol%, acetylene 0.6 mol%, propylene 17 mol%, MA 0.3 mol%, PD 0.3 mol%, with a balance of ethane and propane. The space velocity of the hydrogenation reaction was 15000 h$^{-1}$, the pressure was 2.6 MPa; the inlet temperature of the first stage reactor was 50-60°C, the inlet temperature of the second stage reactor was 50-60°C, and the inlet temperature of the third stage reactor was 55-65°C.

The catalysts prepared in the examples and comparative examples were used in the above-mentioned hydrogenation reaction, and the acetylene content at the outlet of the final reactor was controlled to be less than 0.2 ppm. The results of MAPD conversion (%), operation cycle (h), ethylene selectivity (%), propylene selectivity (%) and C4+ production (mol%) are shown in Table 4.

Among them, the operation cycle refers to the time for which the system can operate when the total acetylene content at the outlet of the final reactor is controlled below 0.2 ppm.

The calculation method for ethylene selectivity is:

$$\text{Ethylene selectivity} = \frac{(C_2H_4)_{\text{outlet}} - (C_2H_4)_{\text{inlet}}}{(\text{acetylene})_{\text{inlet}} - (\text{acetylene})_{\text{outlet}}} \times 100\%.$$

The calculation method for MAPD conversion is:

$$MAPD = \frac{(MAPD)_{outlet} - (MAPD)_{inlet}}{(MAPD)_{inlet}} \times 100\%$$

The calculation method for propylene selectivity is:

$$\text{Propylene selectivity} = \frac{(C_3H_6)_{outlet} - (C_3H_6)_{inlet}}{(MAPD)_{inlet} - (MAPD)_{outlet}} \times 100\%.$$

C4+ production is the sum of the contents of C4 and above components in the component analysis at the outlet of the second-stage reactor.

Table 4. Test results of the catalysts obtained in the examples and comparative examples for hydrogenation of cracked gas stream produced from crude oil

| Catalyst | Operation cycle (h) | Ethylene selectivity (%) | Propylene selectivity (%) | MAPD conversion (%) | C4+ production (mol%) |
|---|---|---|---|---|---|
| A | 1253 | 95 | 100 | 75 | / |
| B | 728 | 85 | 92 | 67 | 0.0008 |
| C | 861 | 88 | 95 | 68 | 0.0005 |
| D | 958 | 90 | 96 | 68 | / |
| E | 1175 | 93 | 99 | 72 | / |
| F | 352 | 54 | 80 | 45 | 0.08 |
| G | 289 | 51 | 78 | 40 | 0.3 |
| H | 427 | 60 | 82 | 51 | 0.01 |
| I | 50 | 35 | 42 | 15 | 0.2 |
| J | 120 | 44 | 68 | 37 | 0.12 |

It can be seen from the results of the selective hydrogenation of the cracked gas stream given in Table 4 that in order to ensure the outlet acetylene of less than 0.2 ppm, the operation cycle, MAPD conversion, ethylene and propylene selectivity and C4+ production of each catalyst are different. Compared with the catalysts in the comparative examples, the catalysts prepared in the examples exhibit longer operation time, better selectivity to ethylene and propylene, higher MAPD conversion and lower C4+ production. This demonstrates that the selective hydrogenation of alkynes and dienes in the cracked gas stream using the catalyst of the present application can effectively remove acetylene under the condition of higher space velocity, obtain higher ethylene selectivity, propylene selectivity and MAPD conversion, and achieve long-term stable operation.

The preferred embodiments of the present application are described in detail above; however, the present application is not limited to the specific details in the above embodiments. Within the technical concept of the present application, a variety of simple modifications can be made to the technical solution of the present application, and these simple modifications all fall within the protection scope of the present application.

It should also be indicated that the various specific technical features described in the above specific embodiments can be combined in any suitable manner without contradiction. In order to avoid unnecessary repetition, various possible combinations will not be further described in the present application.

In addition, various different embodiments of the present application may be arbitrarily combined, and as long as these combinations do not violate the concept of the present application, they should also be regarded as the content invented by the present application.

**Claims**

1. A supported palladium catalyst, comprising a support, and palladium and a modifying component supported on the support, wherein the support is selected from refractory metal oxides, silicon oxide, activated carbon, or combinations thereof, and the modifying component is selected from Bi, Sb, Pb, Sn, Zn, W, Mn, Si, Re, Group VIII elements other than palladium, alkali metal elements, alkaline earth metal elements, Group IIIA elements, Group IB elements, rare earth elements, halogen elements, or combinations thereof, wherein according to an analysis on in-situ infrared spectroscopy of pyrrole adsorption carried out at 40°C, the catalyst exhibits absorption peak(s) in the range of 3251-3410 $cm^{-1}$, preferably in the range of 3350-3400 $cm^{-1}$.

2. The catalyst according to claim 1, wherein according to the analysis on in-situ infrared spectroscopy of pyrrole adsorption carried out at 40°C, the ratio of the peak height of the absorption peak in the range of 3251-3410 $cm^{-1}$ in the in-situ infrared spectrum of the catalyst measured when purging with nitrogen is carried out for 0 minute after 10 minutes of pyrrole adsorption to the peak height of the corresponding absorption peak in the range of 3251-3410 $cm^{-1}$ in the in-situ infrared spectrum measured when purging with nitrogen is carried out for 15 minutes is more than or equal to 5:1, preferably more than or equal to 10:1, for example, 10:1 to 200:1.

3. The catalyst according to any one of the preceding claims, wherein according to the analysis on in-situ infrared spectroscopy of pyrrole adsorption carried out at 40°C, the catalyst exhibits no absorption peak in the range of 3200-3250 $cm^{-1}$, such as in the range of 3160-3250 $cm^{-1}$.

4. The catalyst according to any one of the preceding claims, wherein according to an analysis on in-situ infrared spectroscopy of pyridine temperature-programmed adsorption-desorption, the difference between the surface adsorbed pyridine concentration measured after desorption at 150°C and the surface adsorbed pyridine concentration measured after desorption at 300°C of the catalyst is in the range of 0.1-0.25 mmol/g, preferably in the range of 0.1-0.2 mmol/g, and the surface adsorbed pyridine concentration measured after desorption at 300°C is in the range of 0-0.1 mmol/g, preferably in the range of 0-0.08 mmol/g.

5. The catalyst according to any one of the preceding claims, wherein according to the analysis on in-situ infrared spectroscopy of pyridine temperature-programmed adsorption-desorption, the ratio of the difference between the surface adsorbed pyridine concentration measured after desorption at 150°C and the surface adsorbed pyridine concentration measured after desorption at 300°C to the surface adsorbed pyridine concentration measured after desorption at 150°C of the catalyst is in the range of 60-100%, preferably in the range of 75-100%.

6. The catalyst according to any one of the preceding claims, wherein relative to the weight of the support and on metal element basis, the content of palladium in the catalyst is 0.01-20 wt%, preferably 0.01-5 wt%, more preferably 0.02-1 wt%.

7. The catalyst according to any one of the preceding claims, wherein the catalyst has a specific surface area of 0.5-800 $m^2/g$, preferably 4-200 $m^2/g$, more preferably 15-110 $m^2/g$.

8. The catalyst according to any one of the preceding claims, wherein the support is selected from aluminum oxide, zirconium oxide, gallium oxide, silicon oxide, activated carbon, or combinations thereof; and the modifying component is selected from La, K, Ag, Zn, Ga, Cu, Au, Cs, Bi, Mg, Fe, Re, or combinations thereof.

9. The catalyst according to claim 8, wherein relative to the weight of the support and on metal element basis, the content of the modifying component in the catalyst is 0.01-20 wt%, preferably 0.2-5 wt%.

10. A method for preparing the supported palladium catalyst according to any one of the preceding claims, comprising the following steps:

   1) loading palladium and a modifying component onto a support, followed by drying and calcining to obtain an intermediate; and
   2) subjecting the intermediate to an alkalization treatment with an alkaline solution having a pH value of 7.2-10, preferably 7.5-9, followed by drying and optionally calcining to obtain the catalyst,

   wherein the alkalization treatment is achieved by spraying the alkaline solution, the spraying time is 2-8 min, preferably 3-4 min, and the spraying temperature is 35-70°C, preferably 45-55°C, preferably, the temperature fluctuation during the spraying process is controlled within 5°C, and more preferably, the spraying is constant temperature spraying.

11. The method according to claim 10, wherein:

   in the step 1), the drying is carried out at a temperature of 50-220°C, preferably 80-150°C, for a time of 1-48 h, preferably 2-24 h; the calcining is carried out at a temperature of 300-1500°C, preferably 350-1250°C, for a time of 2-24h, preferably 6-24h, under an atmosphere selected from an inert gas, nitrogen, an oxygen-containing atmosphere, or combinations thereof, wherein the atmosphere for calcining has an oxygen content of 0-40 wt%; and/or

in the step 2), the drying is carried out at a temperature of 60-180°C, preferably 70-140°C, for a time of 1-48 h, preferably 6-20 h; the calcining is carried out at a temperature of 200-500°C, preferably 250-450°C, for a time of 2-24 h, preferably 6-20 h, under an atmosphere selected from an inert gas, nitrogen, an oxygen-containing atmosphere, or combinations thereof, wherein the atmosphere for calcining has an oxygen content of 0-25wt%.

12. The method according to claim 10 or 11, wherein the step 1) comprises loading palladium and the modifying component onto the support together, followed by drying and calcining to obtain the intermediate, wherein in the step 1), the drying is carried out at a temperature of 60-180°C, preferably 80-150 °C, for a time of 1-48 h, preferably 2-24 h; the calcining is carried out at a temperature of 300-700°C, preferably 350-600°C, for a time of 2-24 h, preferably 6-15 h, under an atmosphere selected from an inert gas, nitrogen, an oxygen-containing atmosphere, or combinations thereof, wherein the atmosphere for calcining has an oxygen content of 0-40wt%.

13. The method according to claim 10 or 11, wherein the step 1) further comprises:

1a) loading the modified component onto the support, followed by drying and calcining to obtain a modified support; and

1b) loading palladium and optionally an additional modifying component onto the support, followed by drying and calcining to obtain the intermediate,

preferably:

in the step 1a), the drying is carried out at a temperature of 50-220°C, preferably 80-150°C, for a time of 1-48 h, preferably 2-12 h; the calcining is carried out at a temperature of 700-1500°C, preferably 800-1250°C, for a time of 2-24 h, preferably 6-15 h, under an atmosphere selected from an inert gas, nitrogen, an oxygen-containing atmosphere, or combinations thereof, wherein the atmosphere for calcining has an oxygen content of 0-40wt%; and/or

in the step 1b), the drying is carried out at a temperature of 60-180°C, preferably 80-150°C, for a time of 1-48 h, preferably 2-24 h; the calcining is carried out at a temperature of 300-700°C, preferably 350-650°C, for a time of 2-24 h, preferably 6-15 h, under an atmosphere selected from an inert gas, nitrogen, an oxygen-containing atmosphere, or combinations thereof, wherein the atmosphere for calcining has an oxygen content of 0-40 wt%.

14. The method according to any one of claims 10 to 13, wherein in the step 1), the loading is carried out using a solution containing a precursor of palladium and a solution containing a precursor of the modifying component, and the loading is carried out using a method selected from spraying and impregnation, preferably selected from spraying and incipient wetness impregnation;
preferably:

the solution containing the precursor of palladium used in step 1) has a pH value of 2-5, preferably 3.8-4.5;
in the step 1), relative to the weight of the support and on metal element basis, palladium is used in such an amount that the content of palladium in the catalyst is 0.01-20 wt%, preferably 0.01-5 wt%, more preferably 0.02-1 wt%;
in the step 1), relative to the weight of the support and on metal element basis, the modified component is used in such an amount that the content of the modified component in the catalyst is 0.01-20 wt%, preferably 0.2-5 wt%; and/or
in the step 2), relative to the weight of the support, the alkaline solution comprises an alkaline compound in an amount of 0.1-25 wt%.

15. The method according to claim 14, wherein:

the precursor of palladium is a soluble palladium compound, preferably selected from palladium chloride, palladium nitrate, palladium acetate, palladium sulfate, palladium pivalate, palladium octaethylporphyrin, palladium trimethylacetate, palladium trifluoroacetate, or combinations thereof, more preferably selected from palladium chloride, palladium nitrate, palladium acetate, palladium sulfate, or combinations thereof; and/or
the precursor of the modifying component is a soluble compound containing the modifying component, preferably selected from halides, nitrates, acetates, carbonates, sulfates, hydroxides, amides, compounds containing organic group(s) of the modifying component, or combinations thereof.

16. The method according to any one of claims 10 to 15, wherein:

the alkaline compound comprised in the alkaline solution is an organic alkaline compound, an inorganic alkaline compound or mixtures thereof, wherein the organic alkaline compound is selected from organic amines, pyridine compounds, or combinations thereof, preferably selected from dimethylamine, triethylamine, trifluoroacetamide, pyridine, 4-dimethylaminopyridine, triethanolamine, or combinations thereof; and the inorganic alkaline compound is selected from ammonia water, halides, citrates, oxalates, acetates, bicarbonates, carbonates, or combinations thereof, preferably selected from KF, lithium chloride, potassium acetate, sodium bicarbonate, sodium citrate, or combinations thereof; and/or
the alkaline solution comprises a solvent selected from water, ethanol, acetone, tetrahydrofuran, or combinations thereof;
preferably, the alkaline compound in the alkaline solution has a concentration of 0.1-70 wt%.

17. Use of the supported palladium catalyst according to any one of claims 1 to 9 in hydrogenation of unsaturated hydrocarbon, wherein the unsaturated hydrocarbon is selected from alkynes, polyunsaturated hydrocarbons, or combinations thereof.

18. A method for hydrogenating unsaturated hydrocarbon, comprising a step of contacting an unsaturated hydrocarbon feedstock comprising alkyne and/or polyunsaturated hydrocarbon with the supported palladium catalyst according to any one of claims 1 to 9 in the presence of hydrogen for reaction.

19. The use according to claim 17 or the method according to claim 18, wherein the alkyne is selected from C2-C5 alkynes, preferably selected from C2-C4 alkynes, and the polyunsaturated hydrocarbon is selected from C3-C5 dienes, preferably selected from C3-C4 dienes.

FIG.1A

FIG.1B

FIG.1C

FIG.1D

FIG.1E

FIG.1F

FIG.1G

FIG.1H

FIG.1I

FIG.1J

FIG.2

## INTERNATIONAL SEARCH REPORT

| International application No. |
|---|
| **PCT/CN2023/119966** |

**A. CLASSIFICATION OF SUBJECT MATTER**

B01J23/44(2006.01)i; B01J23/50(2006.01)i; B01J23/54(2006.01)i; B01J23/58(2006.01)i; B01J23/60(2006.01)i; B01J23/62(2006.01)i; B01J37/02(2006.01)i; C07C5/09(2006.01)n; C07C7/167(2006.01)n; C07C11/04(2006.01)n; C07C11/06(2006.01)n

According to International Patent Classification (IPC) or to both national classification and IPC

**B. FIELDS SEARCHED**

Minimum documentation searched (classification system followed by classification symbols)

B01J23; B01J37; C07C5; C07C7; C07C11

Documentation searched other than minimum documentation to the extent that such documents are included in the fields searched

Electronic data base consulted during the international search (name of data base and, where practicable, search terms used)

CNTXT, ENTXTC, VEN, WPABSC, WEB OF SCIENCE, CNKI, 万方, WANFANG: 加氢, 氢化, 丙炔, 丙二烯, 乙炔, 乙烯, 丙烯, 钯, 红外光谱, 碱处理, 碱性溶液, 碱液, 有机胺, 吡啶, 二甲胺, 三乙胺, 三氟乙酰胺, 二甲氨基吡啶, 三乙醇胺, 四甲基乙二胺, 氯化锂, 醋酸钾, 碳酸氢钠, 碳酸氢钾, 柠檬酸钠, 氢氧化钠, 氢氧化钾, 喷涂, 喷淋, 弱碱, MAPD, MA, Pd, KF, NaHCO3, KHCO3, K(OAc), NaOH, KOH, Hydrogenation, alkyne, alkadiene, unsaturated hydrocarbon, Propyne, allene, propylene, palladium, infrared spectrum, alkaline, organic amine, pyridine, dimethylamine, triethylamine, trifluoroacetamide, dimethylaminopyridine, triethanolamine, lithium chloride, potassium acetate, sodium bicarbonate, sodium citrate

**C. DOCUMENTS CONSIDERED TO BE RELEVANT**

| Category* | Citation of document, with indication, where appropriate, of the relevant passages | Relevant to claim No. |
|---|---|---|
| A | US 5583274 A (PHILLIPS PETROLEUM COMPANY) 10 December 1996 (1996-12-10) entire document | 1-19 |
| A | US 4404124 A (PHILLIPS PETROLEUM COMPANY) 13 September 1983 (1983-09-13) entire document | 1-19 |
| A | CN 101875009 A (CHINA PETROLEUM & CHEMICAL CORP. et al.) 03 November 2010 (2010-11-03) entire document | 1-19 |
| A | CN 102247838 A (CHINA PETROLEUM & CHEMICAL CORP. et al.) 23 November 2011 (2011-11-23) entire document | 1-19 |

☑ Further documents are listed in the continuation of Box C.    ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **22 November 2023** | **13 December 2023** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

| International application No. |
|---|
| **PCT/CN2023/119966** |

**C.    DOCUMENTS CONSIDERED TO BE RELEVANT**

| Category* | Citation of document, with indication, where appropriate, of the relevant passages | Relevant to claim No. |
|---|---|---|
| A | CN 102249834 A (CHINA PETROLEUM & CHEMICAL CORP. et al.) 23 November 2011 (2011-11-23)<br>entire document | 1-19 |
| A | CN 102249836 A (CHINA PETROLEUM & CHEMICAL CORP. et al.) 23 November 2011 (2011-11-23)<br>entire document | 1-19 |
| A | CN 105732281 A (CHINA NATIONAL PETROLEUM CORP.) 06 July 2016 (2016-07-06)<br>entire document | 1-19 |
| A | CN 111036200 A (CHINA PETROLEUM & CHEMICAL CORP. et al.) 21 April 2020 (2020-04-21)<br>entire document | 1-19 |
| A | CN 112871198 A (SHANDONG UNIVERSITY) 01 June 2021 (2021-06-01)<br>entire document | 1-19 |
| A | CN 114345336 A (BEIJING UNIVERSITY OF CHEMICAL TECHNOLOGY) 15 April 2022 (2022-04-15)<br>entire document | 1-19 |
| A | US 5587348 A (PHILLIPS PETROLEUM COMPANY) 24 December 1996 (1996-12-24)<br>entire document | 1-19 |

Form PCT/ISA/210 (second sheet) (July 2022)

## INTERNATIONAL SEARCH REPORT
### Information on patent family members

| International application No. |
|---|
| **PCT/CN2023/119966** |

| Patent document cited in search report | | | Publication date (day/month/year) | Patent family member(s) | | | Publication date (day/month/year) |
|---|---|---|---|---|---|---|---|
| US | 5583274 | A | 10 December 1996 | JPH | 08245432 | A | 24 September 1996 |
| | | | | JP | 3866318 | B2 | 10 January 2007 |
| | | | | KR | 960029295 | A | 17 August 1996 |
| | | | | DE | 69633472 | D1 | 04 November 2004 |
| | | | | DE | 69633472 | T2 | 10 November 2005 |
| | | | | DE | 69633472 | T3 | 10 December 2009 |
| | | | | ES | 2224144 | T3 | 01 March 2005 |
| | | | | ES | 2224144 | T5 | 01 May 2009 |
| | | | | EP | 0722776 | A1 | 24 July 1996 |
| | | | | EP | 0722776 | B1 | 29 September 2004 |
| | | | | EP | 0722776 | B2 | 26 November 2008 |
| | | | | ID | 17856 | A | 29 January 1998 |
| | | | | CA | 2164742 | A1 | 21 July 1996 |
| | | | | CA | 2164742 | C | 11 May 1999 |
| | | | | AU | 4098196 | A | 15 August 1996 |
| | | | | AU | 673317 | B2 | 31 October 1996 |
| | | | | US | 5585318 | A | 17 December 1996 |
| | | | | AR | 000764 | A1 | 06 August 1997 |
| US | 4404124 | A | 13 September 1983 | CA | 1172233 | A | 07 August 1984 |
| | | | | DE | 3264885 | D1 | 29 August 1985 |
| | | | | NO | 821487 | L | 08 November 1982 |
| | | | | EP | 0064301 | A1 | 10 November 1982 |
| | | | | EP | 0064301 | B1 | 24 July 1985 |
| CN | 101875009 | A | 03 November 2010 | WO | 2010060281 | A1 | 03 June 2010 |
| | | | | CN | 101733172 | A | 16 June 2010 |
| | | | | CN | 101862653 | A | 20 October 2010 |
| | | | | KR | 20110089357 | A | 05 August 2011 |
| | | | | EP | 2368629 | A1 | 28 September 2011 |
| | | | | US | 2011288353 | A1 | 24 November 2011 |
| | | | | IN | 201101127 | P3 | 30 December 2011 |
| | | | | CN | 101862653 | B | 25 July 2012 |
| | | | | CN | 101875009 | B | 29 August 2012 |
| | | | | EP | 2368629A4 | A4 | 24 October 2012 |
| | | | | RU | 2011126193 | A | 10 January 2013 |
| | | | | RU | 2514438 | C2 | 27 April 2014 |
| | | | | BR | PI0920995 | A2 | 05 January 2016 |
| | | | | KR | 101605055 | B1 | 21 March 2016 |
| | | | | US | 2016136635 | A1 | 19 May 2016 |
| | | | | US | 9643172 | B2 | 09 May 2017 |
| | | | | IN | 294066 | B | 09 March 2018 |
| | | | | BR | PI0920995 | B1 | 13 March 2018 |
| | | | | EP | 2368629 | B1 | 03 April 2019 |
| CN | 102247838 | A | 23 November 2011 | CN | 102247838 | B | 27 March 2013 |
| CN | 102249834 | A | 23 November 2011 | CN | 102249834 | B | 12 March 2014 |
| CN | 102249836 | A | 23 November 2011 | CN | 102249836 | B | 05 February 2014 |
| CN | 105732281 | A | 06 July 2016 | CN | 105732281 | B | 04 September 2018 |
| CN | 111036200 | A | 21 April 2020 | | None | | |
| CN | 112871198 | A | 01 June 2021 | CN | 112871198 | B | 17 May 2022 |
| CN | 114345336 | A | 15 April 2022 | CN | 114345336 | B | 27 December 2022 |

Form PCT/ISA/210 (patent family annex) (July 2022)

## INTERNATIONAL SEARCH REPORT
### Information on patent family members

International application No.

**PCT/CN2023/119966**

| Patent document cited in search report | | | Publication date (day/month/year) | Patent family member(s) | | | Publication date (day/month/year) |
|---|---|---|---|---|---|---|---|
| US | 5587348 | A | 24 December 1996 | SG | 46661 | A1 | 20 February 1998 |
| | | | | JPH | 08290060 | A | 05 November 1996 |
| | | | | AR | 001620 | A1 | 26 November 1997 |
| | | | | ES | 2159658 | T3 | 16 October 2001 |
| | | | | MY | 115733 | A | 30 August 2003 |
| | | | | EP | 0738540 | A1 | 23 October 1996 |
| | | | | EP | 0738540 | B1 | 29 August 2001 |
| | | | | US | 5698752 | A | 16 December 1997 |
| | | | | AU | 5043696 | A | 31 October 1996 |
| | | | | AU | 679873 | B2 | 10 July 1997 |
| | | | | KR | 960037111 | A | 19 November 1996 |
| | | | | KR | 100387206 | B1 | 02 September 2003 |
| | | | | DE | 69614748 | D1 | 04 October 2001 |
| | | | | DE | 69614748 | T2 | 27 June 2002 |
| | | | | CA | 2168387 | A1 | 20 October 1996 |
| | | | | CA | 2168387 | C | 13 July 1999 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 113067000 A

- CN 112517063 A

**Non-patent literature cited in the description**

- Recent Advances in Studying Catalyst Basicity by Infrared Spectroscopy. *Journal of Taiyuan University of Technology*, September 1993, vol. 24, 101-107